Europäisches Patentamt

European Patent Office

Office européen des brevets

(11) Publication number: 0 559 304 A2

# EUROPEAN PATENT APPLICATION

(21) Application number: 93201512.6

(22) Date of filing: 12.06.90

(51) Int. Cl.⁵: C11D 1/83, C11D 3/18, C11D 1/62, C11D 3/20, C11D 3/30, C11D 3/32, C11D 3/00

This application was filed on 26 - 05 - 1993 as a divisional application to the application mentioned under INID code 60.

(30) Priority: 21.06.89 US 369389
09.04.90 US 507335

(43) Date of publication of application:
08.09.93 Bulletin 93/36

(60) Publication number of the earlier application in accordance with Art.76 EPC: 0 410 567

(84) Designated Contracting States:
AT BE CH DE DK ES FR GB IT LI LU NL SE

(71) Applicant: Colgate-Palmolive Company
300 Park Avenue
New York, N.Y. 10022-7499(US)

(72) Inventor: Patel, Amrit M.
35 Wetherhill Way
Dayton, New Jersey(US)
Inventor: Robbins, Clarence R.
1726 Woodfield Road
Martinsville, New Jersey(US)
Inventor: Dixit, Nagaraj S.
3 Titus Lane
Plainsboro, New Jersey(US)
Inventor: Babecki, Raymond E.
5 Evergreen Avenue
Fords, New Jersey(US)

(74) Representative: Kearney, Kevin David Nicholas et al
KILBURN & STRODE 30 John Street
London, WC1N 2DD (GB)

(54) Liquid dishwashing detergent composition.

(57) A liquid dishwashing detergent composition for hand washing of dishes is described, which is mild to human hands and leaves the skin thereof noticeably smoother than conventional and control liquid dishwashing detergent compositions. The invented dishwashing composition includes 5 to 40% of synthetic organic detergent of the anionic and/or nonionic types, which preferably is a mixture of such types, 0.1 to 10% of a skin smoothening compound which is a hydrocarbon, an organic acid, an ester, an amide, an amine, a quaternary ammonium compound, or an alcohol, or any mixture thereof, each of which includes in its formula a hydrocarbyl chain of at least 25 carbon atoms, in an aqueous medium. Preferably, the detergent component is a mixture of sulfated and/or sulfonated anionic detergents, such as linear higher alkylbenzene sulfonate and higher fatty alcohol poly-lower alkoxy sulfate, or is a nonionic detergent or a mixture of a nonionic detergent, such as a condensation product of a higher fatty alcohol and lower alkylene oxide, with a sulfated and/or sulfonated anionic detergent, the skin smoothening compound includes a normal alkane of 25 to 50 carbon atoms and the proportions of synthetic organic detergent, normal alkane skin smoothening compound and aqueous medium are in the ranges of 5 to 35%, 0.2 to 5% and 50 to 80%, respectively.

Also within the invention are a process for hand washing dishes with the invented composition, dissolved in water to form a mild dishwater, which leaves the hands feeling smoother and non-greasy, and a process for manufacturing the invented composition, which latter process is important to produce such compositions having the desired hand smoothening and non-greasy characteristics.

EP 0 559 304 A2

This invention relates to liquid dishwashing detergent compositions. More particularly, it relates to such compositions which are suitable for hand washing of dishes, and which leave the skin of the hands non-greasy and noticeably smoother than it is after washing dishes with ordinary or conventional liquid dishwashing detergent compositions. Additionally, the invention also relates to processes for washing dishes with such detergent compositions and to processes for manufacturing such compositions.

Light duty liquid dishwashing detergent compositions have been and are presently being commercially marketed for use in diluted form in hand washing of dishes or in dishpan dishwashing. Highly alkaline granular dishwashing compositions are often employed for washing dishes in automatic dishwashing machines and recently liquid dishwashing compositions intended for use in automatic dishwashers have also been marketed. Despite the increasing popularity of automatic dishwashers there is still a significant portion of the dishwashing detergent composition market that is held by compositions intended for hand dishwashing applications. Unlike automatic dishwasher detergent compositions which are never in contact with human skin, those intended for hand dishwashing applications should be mild to the skin, and yet must still be effective cleaning agents. This can be difficult to accomplish because immersion of the hands in dishwater removes natural oils from the skin and tends to make it lose its suppleness, so that the hands often may feel dry and hard to the touch. Merely adding emollients to the dishwashing composition has not been found to be a successful solution to this problem because their effects are usually insufficient. Now, however, applicants have discovered that certain types of dishwashing detergent compositions, which are of satisfactory foaming capability, can effectively wash dishes and at the same time leave the hands feeling smooth and non-greasy, which represents a significant advance in the hand dishwashing detergent composition art.

In accordance with the present invention a liquid dishwashing detergent composition for hand washing of dishes, which is mild to human hands and leaves the skin thereof noticeably smoother than a control dishwashing composition, comprises 5 to 40% of synthetic organic detergent selected from the group consisting of synthetic organic anionic detergents preferably a sulfated or sulphonated detergent and synthetic organic nonionic detergents preferably a lower alkoxylated alcohol or phenol, and mixtures thereof, and 0.1 to 10% of a skin smoothening compound (SSC) which is a hydrocarbon, an organic acid, an ester, an amide, an amine, a quaternary ammonium compound, such as a salt, e.g., a chloride, or an alcohol, each of which includes in its formula a hydrocarbyl chain of at least 25 carbon atoms preferably 25 to 50, especially 27 to 39 carbon atoms, or any mixture thereof, in an aqueous medium. The proportions of detergent or soap skin smoothening compound and aqueous medium are preferably 5 to 35%, 0.2 to 5% and 50-80%, respectively. The invention also includes processes for using the compositions of the present invention and for manufacturing them.

A search of prior art patents in US Class 252, subclasses 108, 114, 115, 157, 162, 169, 173, 550, 551 and 558, and in Digests 1, 5 and 14 did not result in the finding of any references which teach the present invention. Among the patents noted in the search were the following:

| | | |
|---|---|---|
| 1,703,602 ; | 4,056,481 ; | 4,247,424 ; |
| 3,708,435 ; | 4,192,761 ; | 4,446,042 ; |
| 3,798,182 ; | 4,228,044 ; | 4,673,525 ; and |
| 4,035,514 ; | | 4,708,813 . |

Some of the mentioned patents refer to incorporating emollients in detergent compositions to make such compositions milder to the human skin, with which it comes into contact during use. Among various adjuvants mentioned in the references are beeswax, paraffin wax, petrolatum, petroleum jelly and micro-crystalline wax. Some of the patents describe the employment of petrolatum and beeswax as additives to detergent compositions for diminishing foaming characteristics of such compositions, and one mentions the use of moisturizers, such as petrolatum and beeswax, in mild skin cleansing compositions.

Although the art indicates that materials which contain compounds that include straight chain alkane groups of 25 carbon atoms or more have been suggested for inclusion in various products, including detergent compositions, nowhere is the importance of the $C_{25}$ normal alkyl moiety mentioned. The art does not disclose nor does it suggest dishwashing detergent compositions containing such compounds, which compositions are satisfactorily foaming, non-greasy and especially mild to the hands. Furthermore, the art does not describe or suggest the use of such compositions and a process for the manufacture thereof, which results in the composition produced being especially effective in smoothening the skin of one who uses it for hand washing of dishes.

References are made herein to compounds and compositions "smoothening" the skin and "softening" the skin. This is not intended to imply that the skin is smoother or softer after use of the compositions of the present invention than it was before but that, as compared with compositions which are otherwise identical apart from the presence of the skin smoothening compound, the skin is left feeling smoother or softer after the use of the composition.

The synthetic organic detergent component of the compositions of the present invention is preferably a synthetic organic anionic detergent or synthetic organic nonionic detergent or a mixture thereof (and of course, mixtures of anionic detergents and mixtures of nonionic detergents may also be employed). Optionally, amphoteric, ampholytic, zwitterionic and even cationic detergents and surface active agents (surfactants) may be present in the invented compositions. Various suitable anionic and nonionic detergents that may be employed are listed in McCutcheon's Detergents and Emulsifiers, North American Edition, 1984, which is incorporated by reference herein. Of those compounds the preferred anionic detergents are of the sulfated and/or sulfonated types, which may be designated as "sulf(on)ated". Such detergents are water soluble salts of a lipophile sulfuric or sulfonic acid. The lipophilic moiety of such acid will normally be of 8 to 30 carbon atoms and will desirably include an alkyl group,

preferably a chain, of 8 or 10 to 18 carbon atoms, more preferably 12 to 14 or 16 carbon atoms, e.g., about 12 carbon atoms. Among such anionic detergents there may be mentioned, as exemplary thereof, the higher alkyl sulfates, the linear higher alkylbenzene sulfonates, the paraffin sulfonates, olefin sulfonates, monoglyceride sulfates and higher fatty alcohol lower alkoxy sulfates. Such higher fatty alcohol or higher alkyl lower alkoxy sulfates are preferably of 10 to 14 or 16 carbon atoms in the higher fatty alcohol or alkyl moiety thereof and of up to 20 moles of lower alkoxy per mole, such as 2 to 20, more preferably 2 to 4 or 6, (with the lower alkoxy normally being ethoxy but sometimes also including up to 30% propoxy). A particularly preferred higher fatty alcohol poly-lower alkoxy sulfate is that wherein the higher fatty alcohol is lauryl alcohol, which is triethoxylated. The anionic detergents are normally employed as water soluble salts of alkali metal, ammonia or alkanolamine, such as triethanolamine. For the most preferred anionic detergents, which are desirably utilized together in applicants' invented anionic compositions, such salts will be sodium salts and the compounds will be sodium linear dodecylbenzene sulfonate and sodium lauryl alcohol triethoxy sulfate.

When the invented detergent compositions are to be nonionic or primarily nonionic, the major detergent component will be a lower alkoxylated alcohol or phenol (the phenol may be substituted with an alkyl group, often a chain of 8 to 10 carbon atoms). The lower alkoxy is normally ethoxy but may also be at least partially propoxy (up to 30% of the alkoxy). Preferably the nonionic detergent will be a condensation product of a higher fatty alcohol of 8 to 15 carbon atoms with 5 to 12 moles of lower alkylene oxide, more preferably with the higher fatty alcohol being of 8 to 12 carbon atoms and condensed with 6 to 10 moles of lower alkylene oxide, and most preferably with the higher fatty alcohol being be of 9 to 11 carbon atoms and condensed with 7 to 9 moles of ethylene oxide, e.g., with 8 moles of ethylene oxide. Similarly, when the ethylene oxide is condensed with alkyl phenol, the moles of ethylene oxide employed will be in similar ranges, per mole of phenol, as per mole of alcohol.

Although dishwashing detergent compositions can be made which utilize only nonionic detergent as the surfactant it is often preferable to incorporate some anionic detergent in such compositions because nonionic detergents are generally low foaming and it is usually desirable to have the liquid dishwashing detergent composition capable of producing foam in the dishwater. While any of the anionic detergents previously mentioned in this specification may be employed in supplementation of the nonionic detergent, it has been found preferable to utilize one which is of a sulfated or sulfonated type and more preferably such is a fatty alkyl sulfate of 8 to 18 or of 10 to 16 carbon atoms, such as ammonium lauryl sulfate.

The "skin smoothening compound" (SSC) of the present compositions, which is so designated for simplicity in view of the unexpectedly beneficial property found by applicants for such group of compounds, is a hydrocarbon, an organic acid, an ester, an amide, an amine, a quaternary ammonium salt or an alcohol, or any mixture thereof, but it is required that such compound employed in the present compositions, to be effective as a non-greasy skin smoothening agent, must include in its formula a hydrocarbyl chain, preferably an alkyl chain, of at least 25 carbon atoms. Preferably, such chain is a normal alkyl or alkylene chain, and is of 25 to 50 carbon atoms in length. It is applicants' discovery that when such a chain is present and is of a length of at least 25 carbon atoms, more preferably 27 to 39 carbon atoms for the n-alkyl or n-alkane thereof, the dishwater, which is made by dilution of the invented composition with water, is capable of smoothening hands that are immersed in it during dishwashing, and accomplishes that without imparting objectionable greasiness to the hands, such as is often associated with uses of other lipophilic compounds. Despite the fact that the dishwater is very dilute with respect to the SSC, in these compositions and in these applications the SSC does deposit on the skin in sufficient quantities to have noticeable and desirable smoothening effects. Such results are surprising in view of the fact that the function of the dishwashing detergent composition is to remove lipophilic materials from surfaces, not to deposit them. Also, it would be expected that the lipophilic SSC would tend to dissolve in the lipophilic phase of any resulting emulsion of oily material from the dishes in the washwater. Contrarily but fortunately, such are not the cases, and although some of the SSC may remain in the wash water, at least a significant proportion thereof deposits on the skin of the hands and smoothens it without making the hands feel greasy. Among the various $C_{25+}$ alkyl-containing compounds that are useful as SSC's in practicing the present invention are pentacosane, heptacosane, nonacosane, melene, cerotic acid, melissic acid, psyllic acid, $c_{26-36}$ alcohols, myricyl palmitate, lacceryl palmitate, myricyl cerotate, myricyl hypogaete, ceryl-2-hydroxypalmitate, $C_{31}$ n-alkane, $C_{39}$ n-alkane, mono- and diamines of the mentioned hydrocarbons, alcohols of the mentioned hydrocarbons and amides of the mentioned acids. As will be seen, the various mentioned SSC's all include at least one normal alkyl or alkylene group of from 25 to 39 carbon atoms, which may be considered as a more preferred range. However, the mentioned compounds are listed as representative and not exclusive. Also, while saturated compounds are preferred, usually in part at least, for stability reasons, unsaturated materials are also useful.

The various SSC's may be employed as pure compounds or in mixtures. They may be synthetic or may be obtained from natural materials. Some have been synthesized and may be employed as the sole SSC in the present compositions or they may be mixed with other such synthesized compounds. Alternatively, such compounds may be obtained from natural materials and products of chemical refineries, such as oil refineries. Such materials and products may be processed to produce higher concentrations of the more desirable SSC's or they may be employed directly. Among some sources of SSC's which are components of the compositions of the present invention are petrolatums, paraffin waxes, beeswax, various waxes from the group of candelilla, Japan, bayberry and montan, and other natural waxes which include the requisite amounts of $C_{25}$ and $C_{25+}$ normal alkyl or normal alkylene-containing compounds. Experimentation has shown that carnauba wax does not produce the desired results. However, micro-crystalline waxes, which include secondary branched chains and primary alicyclic chains, in addition to normal alkanes, have been found to be useful, which apparently indicates that minor branching in a alkyl chain does not seriously interfere with the skin softening function of the $C_{25}$ and $C_{25+}$ alkyl- and alkylene-containing compounds.

The only other required component of the present composition is an aqueous medium, in which the synthetic organic detergent is dissolved and in which the skin smoothening compound is emulsified. Such emulsion, to be effective for the intended purpose, should be made by a certain procedure, which will be described subsequently. The aqueous medium may be water or an aqueous alcoholic medium that may also contain various normal adjuvants, such as are employed in liquid dishwashing detergent compositions. The water utilized is preferably deionized water but tap waters are also satisfactory. The alcohol employed will normally be denatured alcohol, such as SD-40, and when it is present in the liquid dishwashing detergent composition it will usually constitute about 5 to 20% thereof (on the basis of the water and ethanol only), which may be equivalent to about 3 to 15% of ethanol in the final composition. It has been found that it is normally more desirable to employ ethanol in the all-anionic detergent compositions and it has been found that it is sometimes preferable to omit it from the detergent compositions which are primarily based on nonionic detergent.

Among the adjuvants that may be utilized together with the three required components of the invented compositions, in addition to ethanol or other co-solvent, when present, there may be mentioned hydrotropes, detergency improving agents, other surfactants, foaming agents, thickeners, opacifying agents, pearlescing agents, colorants, and perfumes. For example, lauric/myristic monoethanolamide may be employed as a foaming agent (and thickener), sodium cumene sulfonate and sodium xylene sulfonate may be utilized as hydrotropes, and magnesium sulfate may be present to improve detergency (at least for use in areas where the water contains little magnesium ion). Polymeric materials such as hydroxyethyl cellulose, may be employed as thickeners, together with various gums, including carrageenan. Supplemental surfactants, such as cocoamidopropyl betaine may also be employed. Of course, if other properties are desirable in the product the appropriate adjuvants may be included in the formula.

The various proportions of components in the present compositions are important for the obtaining of satisfactory results. Thus, the proportion of synthetic organic detergent will normally be in the range of 5 to 40%, preferably being 5 to 35% and more preferably 15 to 35%. For all-anionic detergent compositions the most preferred range is 25 to 35% of anionic detergent and for compositions which are primarily based on nonionic detergent such more preferred and most preferred ranges are 5 to 30% and 10 to 25%. For the anionic compositions which contain sodium linear dodecylbenzene sulfonate and sodium lauryl alcohol triethoxy sulfate or equivalent detergents the proportion of such sulfonate to such sulfate will usually be within the range of 1:1 to 3:2, and in a most preferred formula there will be present about 17% of sodium linear dodecylbenzene sulfonate and about 13% of sodium lauryl alcohol triethoxy sulfate. For the primarily nonionic detergent compositions the proportion of anionic detergent will preferably be within the range of 2 to 10% and that of the nonionic detergent will be within the range of 8 to 15%, with the proportion of anionic detergent to nonionic detergent being in the range of 2:1 to 8:1. For example, when the nonionic detergent is a condensation product of a higher fatty alcohol of 9 to 11 carbon atoms with 7 to 9 moles of ethylene oxide and the synthetic organic anionic detergent is ammonium fatty alcohol sulfate of 10 to 16 carbon atoms the proportion of the nonionic detergent to the anionic detergent will preferably be within the range of 2:1 to 5:1, as it is in those compositions which contain about 16% of a condensation product of higher fatty alcohol of 9 to 11 carbon atoms with about 8 moles of ethylene oxide, and about 4% of ammonium lauryl sulfate.

The percentage of SSC will normally be within the range of 0.1 to 10% of the composition and preferably will be 0.2 to 5% thereof. More preferably, the normal alkane content of such compound will be 0.2 to 3% of the composition and most preferably it will be 0.5 to 2%. Within the group of SSC's mentioned a sub-group which includes the hydrocarbons, organic acids, alcohols and esters is preferred and within that sub-group it is preferred to employ the hydrocarbons. Such may be obtained from petrolatum, paraffin, beeswax or various other natural materials, refinery products or products of syntheses, providing that such contain the required 25 carbon straight alkyl chain structure. The preferred SSC's, the normal alkanes of 25 to 50 carbon atoms, are readily available from petrolatum and/or beeswax. Petrolatum includes about 60 or 70% of such compounds and beeswax includes about 15% thereof, together with other SSC's. In some formulas, including the preferred formulas of this invention, a preferred proportion of the SSC (preferably n-alkane) is in the range of 0.5 to 1.5%, more preferably 0.5 to 1.0%, e.g., about 0.8% or 0.9%. Such a 0.85% content of the hydrocarbon can be obtained by inclusion of about 1% of the specified type of petrolatum (desirably one which is of a normal distribution curve for carbon chain length, with a peak at about 27 to 33 carbon atoms) and about 1% of beeswax in the formula, but such proportions may desirably vary from 0.1 to 2% of one such component and 0.1 to 2% of the other.

The proportion of aqueous medium will normally be in the range of 45 to 85%, preferably 50 to 80%, for both the anionic and nonionic based compositions. For the anionic compositions a more preferred range is 50 to 70% and still more preferred is 50 to 65%, e.g., about 60% (excluding any ethanol present) and, for the nonionic detergent based compositions the aqueous medium, usually water, will normally be in the range of 60 to 80%, such as about 71%.

The adjuvant contents of the invented detergent composition, excluding ethanol or other solvent, will normally be in the range of 1 to 15% and usually will be in the range of 2 to 10%, such as about 7 or 8%.

Use of the invented composition in dishwashing is a simple matter. All that is required is to fill a sink or dishpan with water, preferably warm or hot water, pour in the desired amount of liquid dishwashing detergent composition, mix it in and add the dirty dishes (preferably after initially scraping and/or rinsing them). The dishes are then rubbed with a cloth, sponge or dishmop to remove remaining food from them, and are rinsed and dried. The amount of dishwashing composition added to the water may be whatever is desired by the user and is considered to be most effective under the circumstances. Usually such proportion will be in the range of 0.1 to 2% of the total dishwater and preferably will be in the range of 0.2 to 1.5%, for example, 0.5 to 1%, but lower proportions can also be effective, especially when only a few dishes are being washed and the soil thereof is relatively light. The temperature of the dishwater is desirably in the range of 30 to 55°C., preferably 35 to 50°C., and the time of immersion of the washer's hands in the dishwater will normally be from 1 or 2 to 5 or 10 minutes. After completion of the washing of the dishes the hands will be rinsed and dried and at that time, after drying, it will be noticed that they are appreciably smoother than after comparable dishwashing operations utilizing control compositions that do not contain the SSC('s) of the invention.

In the process for manufacturing the invented compositions it is important to follow a particular procedure to produce the best and most effective product. Such procedure involves melting the SSC and mixing it with a heated aqueous solution of the synthetic organic detergent, both of which are at about the same elevated temperature, at which the skin smoothening compound and the solution are both in liquid state, such as 75 to 85°C., e.g., about 80°C. In mixing together these components in such manner there results a stable emulsion or microemulsion, which is especially stable in the presence of suitable hydrotrope(s), and which is more effective in smoothening the hands when it is dispersed in the dishwater than are control compositions made by merely mixing together or homogenizing the various components at room temperature, even when the skin smoothening compound is in very finely divided state. While it might be argued that it would be expected that a superior emulsion could be formed by following the invented method, it is surprising that after dispersion in the dishwater there would be an appreciable difference in skin smoothening between the compositions made by the invented process and those that were merely mixed together (or homogenized).

The elevated temperature at which the aqueous detergent solution and melted skin smoothening compound are mixed, in accordance with the invention, will normally be in the range of 70 to 100°C., preferably 75 to 85°C. and after making of the emulsion it will be stirred and cooled to room temperature. It has been found that other lipophilic components of the dishwashing composition may be melted together with the skin softening compound or with a mixture of such compounds, and other water soluble componentS and adjuvants may be dissolved with the detergent component(s) in the aqueous phase, and the desired results are still obtained. The described mixing may be effected by normal mixing equipment, including Lightnin (Registered Trade Mark) mixers, homogenizers, static mixers, in-line mixers, and even conventional stirrers, and the desired emulsions will result. The remaining adjuvants, including any that are temperature sensitive, plus any solvents or other volatiles, such as the ethanol, are gradually added to the cooled emulsion, with stirring, with care being taken so as not to break the emulsion during such additions. Some adjuvants, such as the perfume, may be dissolved in the solvent before such admixing. The products made are stable on storage at room temperature and at elevated temperature, and they leave the hands feeling satisfactorily smooth after dishwashing, without making them feel greasy.

The following examples illustrate but do not limit the invention. Unless otherwise indicated, all parts are by weight and all temperatures are in °C.

EXAMPLE 1

| Component | Percent (by weight) |
|---|---|
| Sodium linear dodecylbenzene sulfonate | 17.0 |
| Sodium lauryl alcohol triethoxy sulfate | 13.0 |
| Lauric/myristic monoethanolamide | 4.0 |
| * Hydrotrope mixture | 3.0 |
| Magnesium sulfate (anhydrous) | 1.0 |
| Beeswax (yellow) | 1.0 |
| ** Petrolatum | 1.0 |
| Perfume | 0.3 |
| Ethyl alcohol (95%) | 5.0 |
| Deionized water | 54.5 |
| | 100.0 |

* Two parts of sodium cumene sulfonate and one part of sodium xylene sulfonate

** Higher molecular weight, with peak distribution for alkyl chain at $C_{27}$, obtainable from Petroleum Industries, Inc.

The liquid dishwashing detergent composition of this formula is made by first dissolving the anionic detergents, hydrotropes and magnesium sulfate in the deionized water, melting the beeswax, petrolatum and lauric/myristic monoethanolamide together by heating to a temperature of about 80°C., heating the aqueous solution to about 80°C., and mixing the solution and melt together with vigorous mixing (using a Lightnin mixer or equivalent) to form a stable emulsion. The emulsion is then cooled to room temperature, which is about 25°C., with stirring, and the perfume, colorant and ethyl alcohol are then admixed with the balance of the formula to produce the final product, which is a stable emulsion.

The liquid dishwashing detergent made is employed in normal manner to hand wash dishes, in both practical use tests and in control tests wherein red dyed beef fat is washed off test plates. In such tests the dishwater is of a mixed calcium and magnesium hardness equivalent to about 150 p.p.m. of $CaCO_3$, its temperature is 41°C. and the concentration of the liquid dishwashing detergent in the dishwater is 1%. The experimental or invented dishwashing detergent was tested against a popular commercial product and also against a control liquid dishwashing composition of the same formula except that the SSC's were omitted and 2% of deionized water was substituted. The invented composition was found to clean the dishes as well as the commercial product and the control, and produced shiny clean dishes, which were not objectionably coated with any greasy or waxy films. Moreover, it was found by the persons running the tests that their hands felt smoother after being immersed in the dishwater during the washings.

To verify the skin smoothening properties of the invented dishwashing composition a panel of six evaluators tested the invented formula and the control for skin smoothening properties. In such test each panelist separately immersed his or her hands in 41°C. dishwaters (before addition of any dishes to them, to avoid any effects of fatty materials from the dishes) for sixty seconds, after which the hands were rinsed under running tap water for sixty seconds and were dried with towels. The observers examined their hands as they dried them, and evaluated the smoothnesses thereof. All six observers found that their hands were smoother after being immersed in the dishwater made from the invented dishwashing composition than when they were immersed in dishwater of the same dilution (1%) made from the control composition.

When the above experiment is repeated, utilizing 0.5% and 1.5% of the dishwashing liquids (invented and control) in the wash water the same superiority in skin smoothening is observed in favor of the invented composition. In further tests, when a twenty-member panel evaluated the same compositions by soaking the hands for ten minutes in 0.1% solutions thereof the invented liquid dishwashing compositions were found to be significantly more effective than the controls in softening the skin of the hands. Because smoothening effects on the skin are very important advantages for hand dishwashing compositions the present results are significant and the advance made in the art by this invention is noteworthy.

EXAMPLE 2

| Component | Percent (by weight) |
|---|---|
| + Neodol (Registered Trade Mark) 91-8 | 16.0 |
| Ammonium lauryl sulfate | 4.0 |
| Cocoamidopropyl betaine | 3.0 |
| Lauric/myristic monoethanolamide | 4.0 |
| Beeswax (yellow) | 1.0 |
| + + Petrolatum | 1.0 |
| Perfume | 0.3 |
| Colorant | 0.2 |
| Deionized water | 70.5 |
| | 100.0 |

+ Condensation product of one mole of fatty alcohol of 9-11 carbon atoms and eight moles of ethylene oxide

+ + Higher molecular weight, with peak distribution for alkyl chain at $C_{27}$

The primarily nonionic dishwashing detergent composition of this example is made in substantially the same manner and is tested in the same way as the composition of Example 1, with the only changes being those necessitated by the different materials being employed. Thus, an aqueous solution of the nonionic detergent and ammonium lauryl sulfate is made and a lipophilic melt is made of petrolatum, beeswax, lauric/myristic monoethanolamide and cocoamidopropyl betaine. The lipophiles are heated to 80°C. and melted and subsequently are mixed with the aqueous phase at 80°C., followed by cooling and addition of the colorant and perfume.

Testings against a popular commercial liquid dishwashing detergent composition and against a control from which the skin smoothening compounds were omitted (replaced by deionized water) are effected in the same manner as described in Example 1 and the same good comparative dishwashing action and improved skin smoothening effects result, as were described in Example 1. Thus, it is seen that the desirable effects of the presences of smoothening SSC's are obtainable with liquid dishwashing detergent compositions of both nonionic and anionic types.

In other comparative experiments, instead of the SSC's of the previous working examples, other lipophilic materials are employed, such as mineral oils and carnauba wax. Such are not effective for softening the hands of one washing dishes in a dishwater containing such dishwashing compositions. It appears that the reasons for this are that mineral oil contains a relatively large percentage of branched chain hydrocarbons and in carnauba wax the major components (esters) include a substantial proportion of aromatic compounds.

EXAMPLE 3

| Component | Percent (by weight) |
|---|---|
| Sodium $C_{12-14}$ alkyl ether ethylenoxy (6 EtO) sulfate | 16.0 |
| Ammonium lauryl sulfate | 6.0 |
| Cocoamidopropyl dimethyl betaine | 4.0 |
| Lauric/myristic monoethanolamide | 3.0 |
| Sodium xylene sulfonate | 1.8 |
| Hydroxyethyl ethylene diamine | 0.2 |
| Beeswax (yellow) | 0.5 |
| + + Petrolatum | 0.5 |
| Ethyl alcohol (95%) | 4.0 |
| Colorant (0.5% aqueous dye solution) | 0.2 |
| Perfume | 0.5 |
| Deionized water | 63.3 |
| | $\overline{100.0}$ |

+ + Higher molecular weight, with peak distribution for alkyl chain at $C_{27}$

EXAMPLE 4

| Component | Percent (by weight) |
|---|---|
| Sodium $C_{12-14}$ alkyl ether ethylenoxy (12 EtO) sulfate | 12.0 |
| Ammonium lauryl sulfate | 6.0 |
| Lauryl dimethylamine oxide | 4.0 |
| Lauric/myristic monoethanolamide | 3.0 |
| Sodium xylene sulfonate | 1.8 |
| Hydroxyethyl ethylene diamine | 0.2 |
| 1-Tricontanol | 1.0 |
| Ethanol (95%) | 4.0 |
| Color (1% aqueous dye solution) | 0.4 |
| Perfume | 0.5 |
| Deionized water | 67.1 |
| | $\overline{100.0}$ |

8

EXAMPLE 5

| Component | Percent (by weight) |
|---|---|
| Sodium linear dodecylbenzene sulfonate | 17.0 |
| Higher alkyl polyglucoside (APG-625) | 17.0 |
| Lauric/myristic monoethanolamide | 2.0 |
| Sodium xylene sulfonate | 3.0 |
| Beeswax, yellow | 1.0 |
| ++ Petrolatum | 1.0 |
| Perfume | 0.3 |
| Colorant (1% aqueous dye solution) | 0.2 |
| Deionized water | 58.5 |
| | 100.0 |

++ Higher molecular weight, with peak distribution for alkyl chain at $C_{27}$

When the liquid dishwashing detergent compositions of Examples 3-5 are made in the manner described in Example 1, with the melted lipophilic phase being admixed with the aqueous phase at elevated temperature (about 80°C.) and then cooled to room temperature with stirring, followed by addition of any heat sensitive components, such as perfume and ethanol, the products resulting are excellent liquid, hand dishwashing detergents which foam satisfactorily, remove greasy soil from dishes without more than light sponging of the dish surfaces, rinse easily from the dishes and leave them shiny clean and with no objectionable cloudy film. Like the products of Examples 1 and 2 the dishwashing detergent compositions of Examples 3-5 also leave the hands of the dish washer feeling smooth, which is attributable to the presence of the SSC('s) therein. Similar results are obtainable when the SSC's of these examples are replaced by long chain length paraffins, such as those averaging in the range of 25 to 39 carbon atoms in straight chain alkanes, by montan wax, candelilla wax, the purified components of beeswax, such as nonacosane, melissic acid, and myricyl cerotate, and n-hentriacontanyl trimethyl ammonium chloride, nonacosyl amine and melissamide, which may be considered as derivatives of such purified components.

Although the embodiments of the present invention primarily described in this specification are liquid dishwashing detergent compositions, in which the surprisingly beneficial skin smoothening effect of the described long chain alkyl containing compounds is very beneficial, in a broader aspect the invention is of skin smoothening detergent compositions which contain such a long chain compound and a synthetic detergent or soap, and which smoothen the skin. Examples 6 and 7 illustrate other such compositions.

EXAMPLE 6

| Component | Percent (by weight) | |
|---|---|---|
| | A | B |
| Sodium laureth sulfate (2 EtO) | 17.0 | 17.0 |
| Coco fatty acids diethanolamide | 6.0 | 6.0 |
| $C_{12-14}$ fatty alcohol | 1.0 | 0.5 |
| Propylene glycol | 0.5 | 0.5 |
| Sodium chloride | 0.5 | 0.5 |
| Citric acid | 1.0 | 1.0 |
| Preservative (Germaben II) | 0.5 | 0.5 |
| Beeswax, yellow | - | 1.0 |
| + + Petrolatum | 1.0 | 1.0 |
| Deionized water | 73.4 | 71.9 |
| | 100.0 | 100.0 |

+ + Higher molecular weight, with peak distribution for alkyl chain at $C_{27}$

The above formula is of a gel type product which is intended for use in the bath or shower to clean both the skin and the hair. In tests similar to those described in Example 1 the experimental formula, 6B, was found by expert evaluators to be significantly better than the formula of Example 6A in smoothening skin and was also found to condition hair better. Such results were confirmed by actual use testing of the products.

EXAMPLE 7

| Component | Percent (by weight) | |
|---|---|---|
| | A | B |
| Sodium alpha $C_{12-14}$ olefin sulfonate | 16.0 | 16.0 |
| Coco fatty acids amidopropyl betaine | 0.8 | 0.8 |
| Coco fatty acids diethanolamide | 5.0 | 5.0 |
| Styrene maleic anhydride copolymer (opacifier) | 0.8 | 0.8 |
| Glycerine | 1.0 | 1.0 |
| Preservative (Germaben II) | 0.5 | 0.5 |
| Sodium chloride | 0.8 | 0.8 |
| Citric acid | 0.2 | 0.2 |
| Perfume | 0.4 | 0.4 |
| + + Petrolatum | - | 1.0 |
| Beeswax | - | 1.0 |
| Deionized water | 74.5 | 72.5 |
| | 100.0 | 100.0 |

+ + Higher molecular weight, with peak distribution for alkyl chain at $C_{27}$

The "liquid soaps" formulations (which are really synthetic organic detergent formulations) of this example are tested in the manner described for the products of Example 6 with essentially the same results. Both the laboratory testing and actual use tests of the formulas of this example prove that Formula 7B, containing the long chain alkyl containing compounds, as such are present in the petrolatum and beeswax, smoothen the hands and condition the hair better than the control compositions of Example 7A.

For the skin smoothening detergent compositions, the broadest proportions of components recited for the liquid dishwashing detergent compositions also apply, 5 to 40% of synthetic organic detergent which is anionic and/or nonionic detergent, and 0.1 to 10% of SSC, in an aqueous medium. For the shower-bath gel formulations and the liquid soap preparations such ranges will normally be 10 to 25%, 0.2 to 3% and 50 to 80%, respectively, more preferably being 10 to 20%, 0.5 to 2% and 65 to 80%, respectively.

EXAMPLE 8

When the invented compositions of the previous examples are modified by substituting for the petrolatum and beeswax components the same total proportion of microcrystalline wax (which is of a molecular weight of about 500) the desired good cleaning effects and skin softening properties are also obtainable, especially if the microcrystalline wax is employed in combination with an equal or greater proportion of the petrolatum and/or beeswax, or active component(s) of such material(s). Similarly, paraffin and paraffin wax may be substituted for the combination of skin softening compounds of the previous examples, providing that alkyl chain lengths thereof are in the required range ($C_{25}$ and higher). In all such cases good cleaning and skin smoothening are obtained and the skin smoothening actions are significantly better than for controls, from the formulas of which such skin smoothening compounds are omitted. Similarly good washing and skin smoothening actions are obtained when other such high molecular weight linear alkyl containing hydrocarbons, esters, "quats", fatty acids, amides, amines and alcohols are substituted for the skin smoothening compounds in the described formulas and in such modifications of such formulas in which other synthetic organic detergents, such as $C_{12-14}$ olefin sulfonates, $C_{14-16}$ paraffin sulfonates, coco monoglyceride sulfates and $C_{8-10}$ alkyl phenol polyethoxylates, are employed. For illustrations, when pentacosane, heptacosane, nonacosane, cerotic acid, melissic acid, psyllic acid, myricyl palmitate, lacceryl palmitate, myricyl cerotate, $C_{31}$ n-alkane, $C_{39}$ n-alkane, $C_{26-36}$ n-alcohols, $C_{26}$ n-alkyl trimethyl ammonium chloride, n-hentriacosyl trimethyl ammonium chloride, amides of the mentioned acids and alcohols and amines of the mentioned hydrocarbons are employed, either singly or in mixture, in replacement of the skin smoothening compounds of the examples, improved skin smoothenings and good cleanings result.

Additionally, the proportions of the detergents, SSC's, adjuvants and aqueous medium may be varied ±10%, ±20% and ±30%, while maintaining them within the ranges heretofore described in this specification, and effective dishwashing detergent compositions, which are significantly better in skin smoothening activity than control compositions which do not contain the skin softening compound(s), are obtained.

Paraffins that may also be utilized will normally be of chain lengths of 20 to 50 carbon atoms, preferably 20 to 40 carbon atoms. Isoparaffins that can be used are preferably of chain lengths in the range of 12 to 16 carbon atoms, preferably 13 to 14 carbon atoms. The petrolatums are desirably petroleum jellies or mineral jellies which melt in the range of 38 to 60°C. The microcrystalline waxes are preferably of an average molecular weight in the range of about 500 to 800 (which is about twice that of the paraffins). $C_{18-36}$ fatty acids and corresponding triglycertides are higher fatty acids and triglycerides which are available from Croda Chemical Corporation (under the tradename Syncrowax HGL-C, for example, for the triglycerides). Stearyl stearate, which is representative of useful esters of both higher fatty alcohols and higher fatty acids, is available from Inolex Corporation, as Lexol SS.

According to the broader aspects of the present invention a detergent or soap composition which is intended for coming into contact with human skin during use thereof, e.g. a dishwashing composition, a shower gel or bath gel, or a "liquid soap" comprises detergent, e.g. synthetic organic detergent, or soap and a compound, herein referred to as a skin smoothening compound, which is a hydrocarbon, an organic acid, an ester, an amide, an amine, a quaternary ammonium compound or an alcohol, or any mixture thereof, the skin smoothening compound including in its formula a hydrocarbyl chain of at least 25 carbon atoms, the composition being such that, in an aqueous medium, skin contacted therewith is leaft feeling smoother, than when contacted with a detergent composition not containing such a skin smoothening compound.

A liquid detergent of the invention preferably comprises synthetic organic detergent selected from the group consisting of synthetic organic anionic detergents and synthetic organic nonionic detergents, and mixtures thereof, preferably 5 to 40% and preferably 0.2 to 10% of the said skin smoothening compound.

The synthetic organic anionic detergent is preferably a sulfated or sulfonates detergent.

The synthetic organic nonionic detergent is preferably a lower alkoxylated alcohol or phenol.

The skin smoothening compound is preferably a hydrocarbon, an organic acid, an ester or an alcohol, or any mixture thereof, each of which contains in its formula an average of 25 to 50 carbon atoms in a linear alkyl chain. The skin smoothening compound is preferably a normal alkane of 25 to 50 carbon atoms.

The proportions of synthetic organic detergent, skin smoothening compound and aqueous medium are preferably in the ranges of 5 to 35%, 0.2 to 5% and 50 to 80%, respectively.

In one form of a liquid detergent composition of the invention the synthetic organic detergent is a synthetic organic anionic detergent or a mixture thereof, the skin smoothening compound includes a normal

EP 0 559 304 A2

alkane of 25 to 50 carbon atoms, and the proportions of synthetic organic anionic detergent, normal alkane skin smoothening compound and aqueous medium are in the ranges of 15 to 35%, 0.2 to 3% and 50 to 70%, respectively, e.g. 25 to 35%, 0.5 to 2% and 50 to 65%, respectively.

The synthetic organic anionic detergent may be a mixture of linear higher alkylbenzene sulphonate, of 10 to 18 carbon atoms in the alkyl thereof, and higher fatty alcohol poly-lower alkoxy sulphate of 10 to 16 carbon atoms in the higher fatty alcohol moiety thereof and of 2 to 20 moles of lower alkoxy per mole.

The normal alkane skin smoothening compound may be in petrolatum and/or beeswax.

In one preferred liquid detergent composition the synthetic organic detergent is a synthetic organic anionic detergent or a mixture thereof, the skin smoothening compound includes a normal alkane of 25 to 50 carbon atoms, and the proportions of synthetic organic anionic detergent, normal alkane skin smoothening compound and aqueous medium are in the ranges of 15 to 35%, 0.2 to 3% and 50 to 70%, respectively. More preferably the synthetic organic anionic detergent may be a mixture of linear higher alkylbenzene sulphonate, of 10 to 18 carbon atoms in the alkyl thereof, and higher fatty alcohol poly-lower alkoxy sulphate of 10 to 16 carbon atoms in the higher fatty alcohol moiety thereof and of 2 to 20 moles of lower alkoxy per mole, the normal alkane skin smoothening compound may be in petrolatum and/or beeswax, and the proportions of synthetic organic anionic detergent, normal alkane skin smoothening compound and aqueous medium are in the ranges of 25 to 35%, 0.5 to 2% and 50 to 65%, respectively.

The synthetic organic anionic detergent mixture may be of sodium linear dodecylbenzene sulphonate and sodium lauryl alcohol triethoxy sulphate, with the proportions thereof preferably being in the range of 1:1 to 3:2 of the sodium linear dodecylbenzene sulphonate to sodium lauryl alcohol triethoxy sulphate.

A specific form of liquid detergent composition of the invention comprises about 17% of sodium linear dodecylbenzene sulphonate, about 13% of sodium lauryl alcohol triethoxy sulphate, about 4% of lauric/myristic monethanolamide, about 2% of sodium cumene sulphonate, about 1% of sodium xylene sulphonate, about 1% of magnesium sulphate, about 1% of petrolatum and about 1% of beeswax, in an aqueous alcoholic medium, which medium contains a minor proportion, in the range of 5 to 20% thereof, of ethanol.

In another form of the invention a major proportion of the synthetic organic detergent is a synthetic organic nonionic detergent or a mixture thereof, the skin smoothening compound includes a normal alkane of 25 to 50 carbon atoms, and the proportions of synthetic organic detergent, normal alkane skin smoothening compound and aqueous medium are in the ranges of 5 to 30%, 0.2 to 3% and 60 to 80%, respectively, e.g. 2 to 10%, 8 to 15%, 0.5 to 2% and 60 to 80%, respectively.

The synthetic organic nonionic detergent may be a condensation product of a higher fatty alcohol of 8 to 15 carbon atoms with 5 to 12 moles of lower alkylene oxide, a synthetic organic anionic detergent may also be present, which is preferably of the sulphated or sulphonated type.

The normal alkane skin smoothening compound may be in petrolatum and/or beeswax.

In another preferred liquid detergent composition a major proportion of the synthetic organic detergent is a synthetic organic nonionic detergent or a mixture thereof, the skin smoothening compound includes a normal alkane of 25 to 50 carbon atoms, and the proportions of synthetic organic detergent, normal alkane skin smoothening compound and aqueous medium are in the ranges of 5 to 30%, 0.2 to 3% and 60 to 80%, respectively, e.g. 2 to 10%, 8 to 15%, 0.5 to 2% and 60 to 80%, respectively. More preferably a synthetic organic anionic detergent is present, which is of the sulphated or sulphonated type, the synthetic organic nonionic detegent is a condensation product of a higher fatty alcohol of 8 to 15 carbon atoms with 5 to 12 moles of lower alkylene oxide, and the normal alkane skin smoothening compound is in petrolatum and/or beeswax.

The synthetic organic nonionic detergent may be a condensation product of a higher fatty alcohol of 8 to 12 carbon atoms with 6 to 10 moles of lower alkylene oxide, the synthetic organic anionic detergent, if present may be higher fatty alkyl sulphate of 8 to 18 carbon atoms.

The proportions of the nonionic detergent to the anionic detergent are preferably in the range of 2:1 to 8:1.

The nonionic detergent may be a condensation product of a high fatty alcohol of 9 to 11 carbon atoms with 7 to 9 moles of ethylene oxide, the synthetic organic anionic detergent, if present, may be an ammonium alkyl sulphate of 10 to 16 carbon atoms. The proportions of the nonionic detergent to the anionic detergent are preferably in the range of 3:1 to 5:1.

Still more preferably the synthetic organic nonionic detergent may be a condensation product of a higher fatty alcohol of 8 to 12 carbon atoms with 6 to 10 moles of lower alkylene oxide, the synthetic organic anionic detergent may be a higher fatty alkyl sulphate of 8 to 18 carbon atoms, and the proportions of the nonionic detergent to the anionic detergent are in the range of 2:1 to 8:1. Alternatively, the nonionic detergent may be a condensation product of a high fatty alcohol of 9 to 11 carbon atoms with 7 to 9 moles of ethylene oxide, the synthetic organic anionic detergent, if present, may be an ammonium alkyl sulphate of 10 to 16 carbon atoms, and the proportion of the nonionic detergent to the anionic detergent is in the range of 3:1 to 5:1.

A specific form of liquid detergent composition comprises about 16% of a condensation product of a higher fatty alcohol of 9 to 11 carbon atoms with about 8 moles of ethylene oxide, about 4% of ammonium lauryl sulphate, about 3% of cocoamidopropyl betaine, about 4% of lauric/myristic monoethanolamide, about 1% of petrolatum and about 1% of beeswax, in about 71% of an aqueous medium.

The invention also extends to a process for manufacturing a liquid detergent composition of the invention which comprises making an aqueous solution of the synthetic organic detergent in an aqueous medium, melting the skin smoothening compound at an elevated temperature, and mixing together the melted skin smoothening compound and the aqueous solution of synthetic organic detergent at about the same elevated temperature.

Preferably the melted skin smoothening compound and the solution of synthetic organic detergent are mixed together at a temperature in the range of 70 to 100°C after which the resulting liquid detergent composition is cooled to room temperature, with stirring during such cooling; more preferably the skin smoothening compound and the aqueous medium are both heated to about 80°C, at which temperature they are both liquid, the skin smoothening compound, in liquid state and at about 80°C is admixed into the aqueous medium also at about such temperature, forming an emulsion, which is then cooled with stirring, to room temperature, after which any heat sensitive components of the liquid detergent composition are admixed into the composition.

The invention has been described with respect to working examples, illustrations and embodiments thereof but is not to be limited to these because it is evident that one of skill in the art, with the present specification before him, will be able to utilize substitutes and equivalents without departing from the invention.

## Claims

1. A detergent or soap composition which is intended for coming into contact with human skin during use thereof, which comprises detergent, e.g. synthetic organic detergent, or soap and a compound, herein referred to as a skin smoothening compound, which is a hydrocarbon, an organic acid, an ester, an amide, an amine, a quaternary ammonium compound or an alcohol, or any mixture thereof, the skin smoothening compound including in its formula a hydrocarbyl chain of at least 25 carbon atoms, the composition being such that, in an aqueous medium, skin contacted therewith is leaft feeling smoother, than when contacted with a detergent composition not containing such a skin smoothening compound.

2. A liquid detergent composition as claimed in claim 1 characterised in that it comprises 5 to 40% of synthetic organic detergent selected from the group consisting of synthetic organic anionic detergents and synthetic organic nonionic detergents, and mixtures thereof, and 0.2 to 10% of the said skin smoothening compound.

3. A liquid detergent composition as claimed in claim 2 characterized in that the synthetic organic anionic detergent is a sulfated or sulfonated detergent and the synthetic organic nonionic detergent is a lower alkoxylated alcohol or phenol, the skin smoothening compound is a hydrocarbon, an organic acid, an ester or an alcohol, or any mixture thereof, each of which contains in its formula an average of 25 to 50 carbon atoms in a linear alkyl chain, and the proportions of synthetic organic detergent, skin smoothening compound and aqueous medium are in the ranges of 5 to 35%, 0.2 to 5% and 50 to 80%, respectively.

4. A liquid detergent composition as claimed in claim 3 characterized in that the syunthetic organic detergent is a synthetic organic anionic detergent or a mixture thereof, the skin smoothening compound includes a normal alkane of 25 to 50 carbon atoms, and the proportions of synthetic organic anionic detergent, normal alkane skin smoothening compound and aqueous medium are in the ranges of 15 to 35%, 0.2 to 3% and 50 to 70%, respectively.

5. A liquid detergent composition as claimed in claim 3 characterized in that a major proportion of the synthetic organic detergent is a synthetic organic nonionic detergent or a mixture thereof, the skin smoothening compound includes a normal alkane of 25 to 50 carbon atoms, and the proportions of synthetic organic detergent, normal alkane skin smoothening compound and aqueous medium are in the ranges of 5 to 30%, 0.2 to 3% and 60 to 80%, respectively.

6. A process for washing dishes which comprises washing such dishes by hand in a aqueous wash water medium containing in the range of 0.1 to 2% preferably 0.2 to 1.5% of a liquid detergent composition as claimed in any one of claims 1 to 5, while immersing the hands in the dishwater, rinsing the hands and drying them, whereby the hands do not feel greasy and are noticeably smoother than after a control process in which dishes are washed in a comparable manner in a control liquid dishwashing detergent composition that does not contain the skin softening compound.

7. A process for manufacturing a liquid detergent composition as claimed in any one of claims 1 to 6 characterized in that it comprises making an aqueous solution of the synthetic organic detergent in an aqueous medium, melting the skin smoothening compound at an elevated temperature, and mixing together the melted skin smoothening compound and the aqueous solution of synthetic organic detergent at about the same elevated temperature.

8. A process as claimed in claim 7 characterized in that the melted skin smoothening compound and the solution of synthetic organic detergent are mixed together at a temperature in the range of 70 to 100ºC after which the resulting liquid detergent composition is cooled to room temperature, with stirring during such cooling.

9. A process as claimed in claim 8 characterized in that the skin smoothening compound and the aqueous medium are both heated to about 80ºC, at which temperature they are both liquid, the skin smoothening compound, in liquid state and at about 80ºC is admixed into the aqueous medium also at about such temperature, forming an emulsion, which is then cooled with stirring, to room temperature, after which any heat sensitive components of the liquid detergent composition are admixed into the composition.